# EUROPÄISCHE PATENTSCHRIFT

(11) **EP 3 746 582 B1**
(45) Veröffentlichungstag und Bekanntmachung des Hinweises auf die Patenterteilung: **22.05.2024**
(21) Anmeldenummer: 19703650.2
(22) Anmeldetag: 28.01.2019
(51) Int. Cl.: C25B 3/20, B01D 9/00, B01D 11/04

(54) **VERFAHREN ZUR ÜBERTRAGUNG EINES ZIELSTOFFS ZWISCHEN ZWEI FLÜSSIGEN PHASEN**
METHOD FOR TRANSFERRING A TARGET SUBSTANCE BETWEEN TWO LIQUID PHASES
PROCÉDÉ DE TRANSFERT D'UNE SUBSTANCE CIBLE ENTRE DEUX PHASES LIQUIDES

(30) Priorität: 29.01.2018 DE 102018000672
(43) Veröffentlichungstag der Anmeldung: 09.12.2020
(73) Patentinhaber: Rheinisch-Westfälische Technische Hochschule Aachen (RWTH), 52062 Aachen (DE)
(72) Erfinder: EGGERT, Armin, 52134 Herzogenrath Nordrhein-Westfalen (DE); GAUSMANN, Marcel, 52074 Aachen (DE); JUPKE, Andreas, 52074 Aachen Nordrhein-Westfalen (DE); MASSMANN, Tim, 49545 Tecklenburg (DE)
(74) Vertreter: Cohausz Hannig Borkowski Wißgott
(86) Internationale Anmeldenummer: PCT/EP2019/051988
(87) Internationale Veröffentlichungsnummer: WO 2019/145531

(56) Entgegenhaltungen:
- US-A- 4 832 813
- US-A1- 2008 047 838
- US-A1- 2011 024 288
- US-A1- 2012 031 769

## Beschreibung

Die Erfindung betrifft ein Verfahren zur Übertragung eines Zielstoffs zwischen zwei flüssigen Phasen gemäß dem Oberbegriff von Anspruch 1. Eine solche Übertragung kann z. B. zum Zweck der Extraktion bzw. Aufreinigung / Aufkonzentrierung des Zielstoffs aus einer der Phasen in eine der Phasen vorgesehen sein. Ein Zielstoff kann z.B. als Atom oder Molekül vorliegen.

Im Stand der Technik sind Extraktionsverfahren bekannt, bei denen zum Zweck der Einstellung für die Extraktion benötigter pH-Werte in einem jeweiligen Prozessschritt Säuren oder Basen zu den Phasen hinzugegeben werden.

Problematisch ist es hierbei, dass dabei mit der Abtrennung eines Zielmoleküls signifikante Mengen Salz entstehen, die ebenso abgetrennt und gegebenenfalls ihrerseits entsorgt werden müssen. Solche bislang im Stand der Technik bekannten Verfahren sind somit gerade aufgrund des Entsorgungsaufwandes häufig unwirtschaftlich. Insbesondere nachgeschaltete Verfahren zur Salzabtrennung, wie beispielsweise Nanofiltration, Umkehrosmose, etc. können häufig nur bis zu einem maximalen Salzgehalt wirtschaftlich betrieben werden, wobei weiterhin auch die Rückgewinnung der eingesetzten Säuren oder Basen aufwendig ist.

Solche bekannten Extraktionsverfahren werden beispielsweise eingesetzt zur Aufreinigung von fermentativ hergestellten Karbonsäuren. Das Verfahren gemäß der Erfindung soll bevorzugt auch in diesem Bereich Anwendung finden, ist jedoch hierauf nicht beschränkt.

Extraktionsverfahren sind z.B. bekannt aus den Dokumenten US 2012/031769 A1, US 2011/024288 A1, US 2008/047838 A1 und US 4 832 813 A.

Vor dem Hintergrund der eingangs genannten Probleme ist es eine Aufgabe der Erfindung, ein Verfahren zur Übertragung eines Zielstoffs zwischen zwei flüssigen Phasen bereitzustellen, insbesondere das zur Extraktion bzw. Aufreinigung oder Aufkonzentrierung eines Zielstoffs, wie beispielsweise einer fermentativ hergestellten Karbonsäure eingesetzt werden kann, bei dem auf den Einsatz von Säuren bzw. Basen zur pH-Wert Anpassung verzichtet werden kann und somit die damit einhergehenden Entsorgungsprobleme entfallen. Es ist weiterhin bevorzugt eine Aufgabe der Erfindung ein Verfahren bereitzustellen, bei dem in einer bevorzugten Ausführung des Verfahrens der Zielstoff, bevorzugt das Zielmolekül auch als Kristallisat gewonnen werden kann.

Erfindungsgemäß wird die Aufgabe gelöst durch ein Verfahren gemäß Anspruch 1. Hierbei ist vorgesehen, dass bei dem Verfahren zur Übertragung eines Zielstoffes, insbesondere eines Zielmoleküls zwischen zwei flüssigen Phasen, wenigstens eine Phase den zu übertragenden Zielstoff umfasst und wenigstens eine Phase eine wässrige Phase ist und wobei zumindest die wässrige Phase in einer von zwei elektrisch leitfähig verbundenen, bevorzugt durch Ladungsträgeraustausch elektrisch leitfähig verbundenen und hinsichtlich ihrer Volumina getrennten Elektrodenkammern angeordnet ist, oder wobei die Phasen gemeinsam in einer von zwei elektrisch leitfähig verbundenen und hinsichtlich ihrer Volumina getrennten Elektrodenkammern angeordnet sind und durch die bei der Elektrolyse entstehenden H- und/oder OH-Ionen in der wässrigen Phase eine pH-Wert-Änderung erzeugt wird, welche einen Übertragungsprozess des Zielstoffes zwischen den Phasen initiiert, wenn sich die Phasen kontaktieren oder in Kontakt gebracht werden.

Die Erfindung kann dabei z.B. vorsehen, nur die wässrige Phase in der Elekrodenkammer anzuordnen und in dieser die pH-Wert-Änderung durch die Elektrolyse durchzuführen, also z.B. in einer Anodenkammer den pH-Wert zu senken oder in einer Kathodenkammer zu erhöhen. Beispielsweise kann erst hiernach diese wässrige Phase mit der anderen Phase in Kontakt gebracht werden, z.B. in der Elektrodenkammer oder auch einer anderen Kammer, wonach der Übertragungsprozess stattfindet. Der Übertragungsprozess ist dabei insoweit durch die pH-Wert-Änderung initiiert bzw. bedingt, als dass die Übertragung des Zielstoffes zwischen den Phasen ohne die pH-Wert-Änderung nicht oder zumindest nur mit geringerer Effizienz stattfinden würde.

Bevorzugt sind beide Phasen gleichzeitig in derselben Elektrodenkammer angeordnet und stehen dauerhaft über ihre Grenzflächen in Kontakt, wobei in der wässrigen Phase die pH-Wert-Änderung erfolgt, was die Übertragung direkt in der Elektrodenkammer initiiert.

Der Begriff der Phase ist in der vorliegenden Beschreibung zu verstehen als eine homogene Materie, die von einer anderen homogenen Materie, insbesondere die der anderen Phase, durch eine Grenzfläche getrennt ist. Die Phasen sind somit ineinander nicht löslich.

Die benannte wässrige Phase ist beispielsweise diejenige, aus welcher heraus der Zielstoff, bevorzugt das Zielmolekül in die andere Phase übergeht, insbesondere was in einer ersten Ausführungsform des Verfahrens vorgesehen ist. Es kann ebenso vorgesehen sein, dass die wässrige Phase diejenige ist, in welche hinein der Zielstoff, bevorzugt das Zielmolekül aus der anderen Phase übergeht. Dies kann insbesondere in einer zweiten Ausführungsform des Verfahrens vorgesehen sein, wie es nachfolgend noch näher beschrieben wird. Die benannte andere Phase ist bevorzugt eine nichtwässrige Phase.

Die benannten Elektrodenkammern bilden insgesamt eine Elektrolyseeinheit, sodass demnach die Elektrodenkammern durch eine Anodenkammer und eine Kathodenkammer gebildet werden. Jede Kammer umfasst ein inneres Volumen, in welchem die beiden Phasen aufgenommen sind. Die Phasen können in die Kammer eingefüllt sein oder diese durchfließen. Insbesondere ist somit das Verfahren batch-weise oder auch kontinuierlich betreibbar. Weiterhin umfasst jede Kammer eine Elektrode, nämlich eine Anode oder Kathode.

Die Trennung der Volumina bei den Elektrodenkammern bedeutet, dass sich die darin jeweils befindlichen Inhalte, insbesondere also die Phasen an denen das Verfahren durchgeführt wird, sich nicht zwischen den Elektrodenkammern austauschen können. Sehr wohl sind die Elektrodenkammern jedoch leitfähig verbunden, damit die Elektrolyse stattfinden kann.

Die Elektrodenkammern sind bevorzugt so elektrisch leitend miteinander verbunden, dass während der Elektrolyse ein Ladungsträgerausgleich zwischen den Kammern stattfinden kann. Die Trennung der Kammern erfolgt beispielsweise durch eine semipermeable Kammerwand (Diaphragma) oder eine semipermeable Membran, welche für die Phasen undurchlässig ist, aber zumindest Ladungsträger passieren lässt. Vorzugsweise wird die Trennung so ausgeführt, dass kein diffusiver oder konvektiver Transport des Zielstoffs/- moleküls entgegen dem gewünschten Konzentrationsgradienten stattfindet, insbesondere wobei der Ladungsausgleich durch andere in den Phasen befindliche Ladungsträger erfolgt.

Wird somit die Übertragung des Zielstoffs zwischen zwei Phasen in einer der Elektrodenkammern betrachtet, so weist die andere an der Elektrolyse beteiligte Elektrodenkammer einen Elektrolyten auf, insbesondere im einfachsten Fall Wasser auf oder z.B. auch die wässrige Phase von den beiden beteiligten Phasen.

Der benannte Übertragungsprozess zur Übertragung des Zielstoffes zwischen den beiden Phasen kann z.B. ein Prozess sein, bei dem der Zielstoff aus der einen Phase heraus und in der anderen Phase in Lösung geht.

Beispielsweise kann die Erfindung vorsehen, dass durch die zwischen den Elektrodenkammern stattfindende wässrige Elektrolyse der Zielstoff durch die bei der Elektrolyse entstehenden H-Ionen protoniert oder durch die bei der Elektrolyse entstehenden OH-Ionen deprotoniert wird, wobei der Übertragungsprozess gebildet wird durch das Lösen des Zielstoffes in derjenigen der beiden Phasen, in welcher der durch die Elektrolyse protonierte Zielstoff oder der durch die Elektrolyse deprotonierte Zielstoff die höhere Löslichkeit aufweist. Durch den Wechsel des Protonierungszustandes des Zielstoffes bei der Elektrolyse wird somit die Löslichkeit des Zielstoffes in der einen Phase, in welcher er sich zunächst befindet, herabgesetzt und die Löslichkeit in der anderen Phase, in die er übergeht, heraufgesetzt.

Ebenso kann die Erfindung vorsehen, dass durch die zwischen den Elektrodenkammern stattfindende wässrige Elektrolyse durch die entstehenden H+ und/oder OH- Ionen die Bindung des Zielstoffs an einen Bindungspartner in einer der Phasen oder die Abspaltung des Zielstoffs von einem Bindungspartner in einer der Phasen initiiert wird, insbesondere durch pH-Wert-abhängige Aktivierung des Bindungspartners und/oder durch eine Protonierung / Deprotonierung des Zielstoffs. Beispielsweise kann die Bindung an den Bindungspartner mit einer Protonierung des Zielstoffes einhergehen und/oder die Abspaltung von einem Bindungspartner mit einer Deprotonierung des Zielstoffes einhergehen.

Hierbei kann die Erfindung z.B. vorsehen, dass in beiden Elektrodenkammern einer Elektrolyseeinheit die zueinander konträren Protonenreaktionen erzeugt werden. Das heißt, in einer der Kammern, der Anodenkammer, findet eine Protonierung eines Zielmoleküls in einer Phase statt, während gleichzeitig in einer anderen Elektrodenkammer, der Kathodenkammer, eine Deprotonierung insbesondere desselben Zielstoffs stattfindet. Dies ist jedoch für die Erfindung nicht zwingend.

In beispielhafter Ausführung kann z.B. eine Protonenproduktion und durch Protonen vermittelte Bildung der Bindung zum Bindungspartner in der einen Elektrodenkammer mit der Hydroxidionenproduktion und OH- initiierten Spaltung der Bindung zwischen Zielstoff und Bindungspartner in der anderen Kammer elektrisch verschaltet werden. Es wäre auch möglich, nur eine Reaktion zu nutzen und als Gegenreaktion eine andere Halbzellenreaktion zu nutzen (z.b. Produktion einer Base, z.B. zur Neutralisation eines sauren Rückstroms einer Phase aus der Anodenkammer)

Wesentlich ist für das weitere Verständnis, dass der jeweils betrachtete Zielstoff, insbesondere das jeweils betrachtete Molekül sowohl in seinem protonierten als auch im deprotonierten Zustand als Zielstoff /Zielmolekül bezeichnet und verstanden wird.

Der wesentliche Kerngedanke des Verfahrens beruht darauf, dass durch die Wasserelektrolyse in den räumlich getrennten Kammern der Elektrolyseeinheit H⁺- bzw. OH⁻- Ionen erzeugt werden, was eine Änderung des pH-Wertes der wässrigen Phase bewirkt. Hierdurch kann die erforderliche Einstellung des pH-Wertes durch den direkten Einsatz von Strom erfolgen. Eine externe Säure- oder auch Basezugabe wird hierdurch überflüssig und eine damit einhergehende Salzentstehung vermieden. Die Abtrennung des Zielstoffs erfolgt hier demnach durch eine pH-abhängige bzw. Säure/Base katalysierte Extraktion. Gemäß später erfolgender Beschreibung kann die Erfindung auch vorsehen, einen pH-abhängigen Kristallisationsschritt anschließen zu lassen, der ebenfalls in einer Elektrodenkammer durchgeführt werden kann.

Bei der Übertragung des Zielstoffs, bevorzugt Zielmoleküls zwischen den Phasen, in zumindest einer der Elektrodenkammern sieht die Erfindung bevorzugt in allen möglichen Ausführungen vor, dass die Phasen in der Elektrodenkammer während der Durchführung der Elektrolyse dispergiert sind.

Beispielsweise kann hierfür in der Elektrodenkammer aktiv die Dispersion betrieben werden, z. B. durch ein Durchmischen der Phasen mittels bewegten Mischelementen wie beispielsweise einem Rührer. Ebenso kann die Dispersion durch Ultraschall erzeugt werden, insbesondere mit einem Ultraschall erzeugenden Element innerhalb der Elektrodenkammer.

Die Phasen können auch ausserhalb der Elektrodenkammer dispergiert werden und im dispergierten Zustand in die Kammer eingebracht werden. Es kann vorgesehen sein, dass die Dispersion durch die Strömungen innerhalb der Kammer erzeugt oder eine vorher erzeugte Dispersion erhalten bleibt, z.B. durch die Strömung, welche durch die aufsteigenden Gasblasen erzeugt wird, ggfs. unter Einsatz von passiven strömungsleitenden Elementen in der Kammer.

Eine solche Dispersion wird bevorzugt bei allen möglichen Verfahrensschritten betrieben, in denen eine Übertragung zwischen zwei flüssigen Phasen stattfindet.

Durch das Durchmischen und erzielen der Dispersion wird eine erhebliche Vergrößerung der Grenzfläche zwischen den Phasen bereitgestellt, sodass der Transfer des Zielstoffs/Zielmoleküls zwischen den Phasen mit einer höheren Effizienz ermöglicht wird.

Die Erfindung kann vorsehen, dass die Phase, in welche der Zielstoff übergeht ein organisches Lösungsmittel umfasst, insbesondere diese Phase durch das organische Lösungsmittel gebildet ist. Ein solches organisches Lösungsmittel kann z.B. gewählt sein aus Alkoholen z.B. Octanol, Estern z.B. Butylacetat oder aliphatischen Verbindungen.

Diese Phase kann alternativ oder zusätzlich einen pH-Wert abhängig reaktiven Bindungspartner umfassen oder durch diesen ausgebildet sein. Dieser kann z.B. ausgewählt sein, aus der Gruppe der aliphatischen Amine mit wenigstens 10 Kohlenstoffatomen oder Organophosphorsäuren mit 2 bis 3 Alkylgruppen..

Der Zielstoff ist bevorzugt ein Zielmolekül, vorzugsweise, welches wenigstens eine funktionelle Gruppe besitzt, die dem Stoff eine pH-Wert abhängige Löslichkeit verleiht, und/oder die pH-Wert abhängig an reversiblen Reaktionen teilnehmen kann z.B. eine Carboxylgruppe. Das Zielmolekül kann z.B. ausgewählt sein aus der Gruppe der wasserlöslichen Aminosäuren und / oder wasserlöslichen Carbonsäuren.

Eine bevorzugte Ausführung der Erfindung kann vorsehen, dass eine erste Phase durch eine wässrige Lösung einer Säure als Zielmolekül gebildet wird und eine zweite Phase entweder ein Reaktivextraktionsmittel als Bindungspartner umfasst, insbesondere diese Phase aus diesem Reaktivextraktionsmittel gebildet wird, und/oder die zweite Phase ein organisches Lösungsmittel umfasst, insbesondere aus diesem gebildet wird wobei in der Anodenkammer von beiden Elektrodenkammern die Säure protoniert wird und wobei die Säure reaktiv an das Reaktivextraktionsmittel angelagert wird, insbesondere durch Wasserstoffbrückenbindungen, oder die Säure in das organische Lösungsmittel in Lösung geht, in welchem die protonierte Säure eine höhere Löslichkeit hat im Vergleich zur deprotonierten Säure.

Bei der Bindung der Säure an das Reaktivextraktionsmittel kann ein Komplex gebildet werden, bevorzugt, welcher in der zweiten Phase in Lösung geht.

In beiden möglichen Fällen wird demnach die Säure von der ersten Phase in die zweite Phase übertragen.

Bevorzugt hat dabei die zweite Phase ein geringeres Volumen in der Anodenkammer als die erste Phase. Bevorzugt kann bereits dies zu einer Aufkonzentrierung bzw. Aufreinigung des Zielmoleküls in der zweiten Phase gegenüber der ersten Phase führen, dies ist jedoch für die Erfindung nicht zwingend.

Durch die Wahl und Einstellung verschiedener pH-Werte können verschiedene Säuren in Abhängigkeit ihres pKs Wertes selektiv voneinander getrennt werden. Die Einstellung des für eine bestimmte Säure nötigen pH-Wertes kann z.B. durch die Anpassung von Elektrolyseleistung und/oder Verweilzeit realisiert werden.

Besonders bevorzugt ist es hier vorgesehen, dass die erste Phase einem Fermentationsreaktor entnommen wird, insbesondere somit diese erste Phase eine Säure in wässriger Lösung umfasst, die durch Fermentation gewonnen ist.

Besonders bevorzugt kann das Verfahren weiterhin vorsehen, dass nach der Durchführung der Übertragung des Zielmoleküls auf die zweite Phase gemäß den vorgenannten Schritten die erste Phase einer Kathodenkammer zugeführt wird, in der ebenso eine Elektrolyse durchgeführt wird, diese erste Phase aus der die Säure extrahiert wurde durch die Elektrolyse behandelt wird und in den Fermentationsreaktor hiernach zurückgeführt wird. Beispielsweise kann die Zurückführung in den Fermentationsreaktor während der Durchführung der Elektrolyse oder auch danach vorgenommen werden. Auch diese Behandlung kann batchweise oder kontinuierlich erfolgen, jeweils z.B. in von der wässrigen Phase in Reihe durchflossenen Kammern einer Elektrolyseeinheit.

Durch die Elektrolyse in der Kathodenkammer wird der bei der Extraktion abgesenkte pH-Wert der ersten Phase wieder angehoben. Bevorzugter Weise kann die benannte Kathodenkammer eine solche sein, die elektrisch zu der Anodenkammer zugeordnet ist, bei der das vorbenannte Verfahren durchgeführt wird. Dabei bildet immer eine Kathodenkammer und Anodenkammer eine Elektrolyseeinheit.

Eine andere zweite Ausführungsvariante des Verfahrens kann es vorsehen, dass eine erste Phase durch einen Komplex eines Reaktivextraktionsmittels als Bindungspartner und einer daran, insbesondere durch Wasserstoffbrückenbindungen, gebundene protonierte Säure als Zielmolekül gebildet wird oder eine erste Phase durch ein organisches Lösungsmittel gebildet wird, in welchem eine protonierte Säure gelöst ist und die zweite Phase Wasser umfasst, wobei in der Kathodenkammer von beiden Elektrodenkammern die protonierte Säure deprotoniert wird und entweder durch das Aufbrechen der Bindungen, insbesondere der Wasserstoffbrückenbindungen vom Reaktivextraktionsmittel abgespalten wird, bevorzugt dabei in der wässrigen Phase in Lösung geht oder, sofern kein Reaktivextraktionsmittel vorliegt die deprotonierte Säure in der wässrigen Phase in Lösung geht. Somit wird hier durch Dissoziation (von dem Reaktivextraktionsmittel) oder nur durch Lösung die Säure von der ersten Phase in die zweite Phase übertragen.

Diese Übertragung kann insbesondere dadurch erleichtert sein, dass die deprotonierte Säure in der zweiten wässrigen Phase eine höhere Löslichkeit aufweist, im Vergleich zur protonierten Säure. So wird demnach mit diesem Verfahrensschritt das Zielmolekül in eine wässrige Phase übertragen und hierbei bevorzugt aufgereinigt bzw. weiter konzentriert.

Bevorzugt hat dabei die zweite Phase auch in diesem Verfahrensschritt ein geringeres Volumen in der Kathodenkammer als die erste Phase. Bevorzugt kann bereits dies zu einer Aufkonzentrierung bzw. Aufreinigung des Zielmoleküls in der zweiten Phase gegenüber der ersten Phase führen, dies ist jedoch für die Erfindung nicht zwingend.

Die Erfindung sieht es vor, dass die zuvor benannte zweite Ausführungsform des erfindungsgemäßen Verfahrens der zuvor benannten ersten Ausführungsform des erfindungsgemäßen Verfahrens zeitlich nachgeschaltet ist oder beide gleichzeitig durchgeführt werden, wobei die zweite Phase des einen, insbesondere des die Protonierung durchführenden Verfahrensschrittes die erste Phase des anderen, insbesondere des die Deprotonierung durchführenden Verfahrensschrittes bildet.

So wird demnach bei dieser erfindungsgemäßen Ausführung dieses zweistufigen bzw. parallelen Verfahrens das (zunächst) in wässriger Lösung vorliegende Zielmolekül, insbesondere durch die Protonierung in der Anodenkammer auf das benannte Reaktivextraktionsmittel als Bindungspartner, z.B. durch das Entstehen von Wasserstoffbrückenbindungen übertragen und anschließend in eine wässrige Phase rückgeführt. Dies wird wie eingangs beschrieben bevorzugt in einem dispergierten Zustand der beiden Phasen vorgenommen.

Die Erfindung kann vorsehen, dass das Absetzen der beiden Phasen abgewartet wird, um diese sodann zu trennen. Die zweite Phase, insbesondere der gebildete Komplex von Reaktivextraktionsmittel und Zielmolekül kann sodann als separierte Phase aus der Anodenkammer in die Kathodenkammer zur Durchführung des zweiten Verfahrensschrittes übertragen werden, insbesondere wobei sodann durch die bei der Elektrolyse stattfindende Aufspaltung der Wasserstoffbrückenbindungen und Deprotonierung, insbesondere unter den vorbenannten unterschiedlichen Löslichkeitsverhältnissen, sodann das deprotonierte Zielmolekül in die Wasserphase übertragen wird. Vor der Durchführung der Deprotonierung in der Kathodenkammer kann die Anodenkammer bereits wieder mit zwei Phasen befüllt werden, so dass Extraktion (z.B. unter Protonierung) und Rückextraktion (z.B. unter Deprotonierung ) zweitgleich erfolgen können.

Die Hintereinanderschaltung der vorbeschriebenen Verfahrensschritte bewirkt somit, dass das Zielmolekül aus einer wässrigen Phase wiederrum in eine wässrige Phase, bevorzugt unter Erhöhung der Konzentration in der letzten wässrigen Phase überführt wird.

Eine bevorzugte Weiterbildung dieses zweistufigen Verfahrens, aber auch bereits alleine der vorbenannten zweiten Ausführungsform des Verfahrens, in welcher eine Übertragung in eine wässrige Phase durchgeführt wird, kann vorsehen, dass die zweite Phase, insbesondere also die wässrige Phase in die das Zielmolekül in dem vorbenannten Deprotonierungsschritt übertragen wurde, in eine Anodenkammer einer Elektrolyseeinheit überführt wird.

Hierbei kann es sich um die Anodenkammer einer beliebigen Elektrolyseeinheit handeln, bevorzugt jedoch um die Anodenkammer, die derjenigen Kathodenkammer elektrisch zugeordnet ist, in welcher der zuvor durchgeführte Deprotonierungsschritt stattfindet.

Es ist so dann vorgesehen, durch Senkung des pH-Wertes in der zweiten wässrigen Phase mittels der durchgeführten Elektrolyse aufgrund der pH-Wert abhängigen Löslichkeit der Säure in Wasser, die Säure auszukristallisieren. Die Erfindung kann weiter vorsehen, dass die von der auskristallisierten Säure separierte zweite Phase in diejenige Kathodenkammer zurückgeführt wird, in welcher der Deprotonierungsschritt stattfindet. So kann beispielsweise die zweite Phase im Umlauf zwischen diesen Elektrodenkammern geführt werden. Statt mit der benannten Säure kann der Schritt der Auskristallisation auch mit anderen Zielmolekülen erfolgen.

Eine darüber hinausgehend bevorzugte Ausführungsform kann es vorsehen, dass die vorbenannte Anodenkammer und die Kathodenkammer, die zu den beiden aufeinanderfolgenden oder parallel durchgeführten Verfahrensschritten der Protonierung und Deprotonierung erwähnt sind, die Elektrodenkammern von verschiedenen Elektrolyseeinheiten sind.

So besteht hier beispielsweise die Möglichkeit, dass durch die Anodenkammer einer ersten Elektrolyseeinheit der vorbeschriebene Verfahrensschritt der pH-Wert-abhängigen Übertragung (z.B. mit Protonierung) des Zielstoffs durchgeführt wird, wobei in der Kathodenkammer dieser Elektrolyseeinheit eine pH-Wert Anpassung der ersten Phase, aus welcher heraus der Zielstoff extrahiert wurde, vorgenommen wird, bevor diese erste Phase weiter verwendet wird, z.B. die erste Phase, die aus einem Fermentationsreaktor entnommen wurde, in diesen zurückgeführt wird.

So besteht demnach hierdurch die Möglichkeit, den pH-Wert der nach der Extraktion zurückbleibenden ersten Phase wieder an seinen ursprünglichen pH-Wert anzupassen, den diese erste Phase hatte, als sie aus dem Fermentationsreaktor zum Zweck der Extraktion entnommen wurde oder diesen auch zu erhöhen.

Die vorbenannte Rückextraktion des Zielstoffs aus dem gebundenen Komplex oder der organischen Lösung zurück in die wässrige Phase gemäß dem vorbeschriebenen zweiten Verfahrensschritt kann somit mit einer Kathodenkammer einer zweiten Elektrolyseeinheit vorgenommen werden. In der Anodenkammer dieser Elektrolyseeinheit kann sodann bevorzugt der Auskristallisationsschritt vorgenommen werden, der ebenso zuvor beschrieben wurde. Der auskristallisierte Feststoff wird sodann von der wässrigen Phase getrennt, bevorzugt die wässrige Phase in einen Verfahrenskreislauf rückgeführt.

Eine mögliche Ausführung kann auch vorsehen, dass die zuvor beschriebene erste Elektrolyseeinheit entfällt, beispielsweise, wenn eine Fermentation in einem Fermentationsreaktor bereits bei einem pH-Wert betrieben wird, in der das Zielmolekül in protonierter Form vorliegt. Die Fermentationsbrühe eines so betriebenen Fermentationsreaktors kann direkt eine erste wässrige Phase bilden, aus der das Zielmolekül wie zuvor beschrieben durch Lösung oder reaktiv extrahiert wird. Es bedarf somit keiner Elektrolyse zur pH-Wert-Anpassung. Die Erfindung kann in diesem Fall z.B. nur eine Elektrolyseeinheit vorsehen, in deren Kathodenkammer die Rückextraktion gemäß der vorherigen Beschreibung zur zweiten Ausführung betrieben wird und in deren Anodenkammer eine Auskristallisation betrieben wird gemäß der vorbeschriebenen Weiterbildung der zweiten Ausführung.

Bei den jeweils durchgeführten Elektrolysen entstehen zwangsläufig Sauerstoff sowie auch Wasserstoff. Bevorzugter Weise sieht es hier die Erfindung vor, den Sauerstoff der bei der Elektrolyse entsteht für die Sauerstoffversorgung eines Fermentationsreaktors zu nutzen.

Allgemein kann es jedoch vorgesehen sein, die entstehenden beiden Gase zu recyceln, durch Zuführung in eine entsprechende Nutzungsmöglichkeit, z.B. eine energetische Verwertung oder Einsatz in Hydrierungs-/ Hydroformulierungsschritten in weiteren Reaktionsschritten des Prozesses. Ebenso können die Gase in Druckbehältern gespeichert werden.

Allgemein ist nach einer bevorzugten Ausführungsform der Erfindung das Zielmolekül eine gesättigte oder ungesättigte Carbonsäure mit wenigstens einer Carboxylgruppe, die ggfs. substituiert ist, bevorzugt mit sauerstoffhaltigen funktionellen Gruppen. Dies kann eine Alkansäure mit 1 bis 4 Kohlenstoffatomen, beispielsweise Essigsäure oder Buttersäure, und /oder eine Alkandisäure mit 2 bis 6 Kohlenstoffatomen, beispielsweise Oxalsäure, Malonsäure, Bernsteinsäure, Adipinsäure sein. Weiter kann dies eine (Poly)hydroxyalkansäure mit 2 bis 6 Kohlenstoffatomen, beispielsweise Glykolsäure, Milchsäure oder Glukonsäure, und / oder eine ungesättigte Alkandisäure mit 2 bis 6 Kohlenstoffatomen, beispielweise Itaconsäure, Muconsäure sein. Weiterhin kann dies eine Alkansäure mit 4 bis 6 Kohlenstoffatomen mit wenigstens 2 Carboxlgruppen und wenigstens 1 Hydroxylgruppe, beispielsweise Weinsäure oder Zitronensäure sein. Bevorzugt ist es, als wasserlösliche Carbonsäure die bei Temperaturen von 20 °C festen Carbonsäuren zu verwenden, die bei der Rückextraktion durch gezielte pH-Wert Einstellung ausgefällt werden können, bevorzugt in kristalliner Form. Hierzu eignet sich insbesondere Itaconsäure und / oder Bernsteinsäure und oder Gemische, die wenigstens eine dieser Carbonsäuren enthalten.

Nach einer weiteren bevorzugten Ausführungsform der Erfindung ist das Zielmolekül eine wasserlösliche Aminosäure, die ausgewählt ist aus nichtproteinogenen und / oder proteinogenen Aminosäuren

Die vorgenannten wasserlöslichen Aminosäuren, und / oder wasserlöslichen Carbonsäuren liegen im Zielmolekül entweder einzeln oder als Gemisch, bevorzugt als Gemisch vor.

Nach einer bevorzugten Ausführungsform der Erfindung ist der Bindungspartner ein aliphatisches Amin mit wenigstens 10 Kohlenstoffatomen, welches primär, sekundär oder tertiär ist, wobei der oder die Alkylreste vorzugsweise insgesamt 20 bis 50 Kohlenstoffatome aufweisen, und diese linear, cyclisch oder verzweigt sind, beispielsweise Tributylamin, Trioctylamin, Tridodecylamin und / oder Triocylamin-1-octanol, Bevorzugt ist Tributylamin, Trioctylamin und / oder Triocylamin-1-octanol.

Nach einer weiteren bevorzugten Ausführungsform der Erfindung ist der Bindungspartner eine Organophosphorsäure in Form von linearen oder verzweigten Alkylphosphaten mit 2 bis 3 Alkylgruppen, wobei jede Alkylgruppe 1 bis 10 Kohlenstoffatome aufweist, beispielsweise Tributylphosphat und / oder Di-(2-ethylhexyl)phosphorsäure. Bevorzugt ist Tributylphosphat (TBP) und / oder Di-(2-ethylhexyl)phosphorsäure (D2EHPA).

Nach einer weiteren bevorzugten Ausführungsform der Erfindung ist der Bindungspartner eine quartäre Ammoniumverbindungen mit wenigstens 10 Kohlenstoffatomen, beispielsweise Cetyltriethylammonium (CTAB) und / oder, Tetrabutylammoniumhydroxid (TBAH).

Die vorgenannten aliphatischen Amine mit wenigstens 10 Kohlenstoffatomen, Organophosphorsäuren mit 2 bis 3 Alkylgruppen, quartären Ammoniumsalze , liegen im Bindungspartner entweder einzeln oder als Gemisch, bevorzugt als Gemisch vor.

Das erfindungsgemäße Verfahren erschließt es somit besonders bevorzugt fermentativ hergestellte Carbonsäuren aufzureinigen bzw. zu konzentrieren, ist jedoch auf diese Anwendung nicht beschränkt. Weitere Anwendungen sind die pH-sensitive Extraktion von Aminosäuren, Aminen oder anderen Ionen, deren Extrahierbarkeit vom pH-Wert abhängig ist, z.B. eine Seitengruppe, die an einer Gleichgewichtsreaktion ph-abhängig teilnehmen kann: z.B. Carboxygruppe, Hydroxygruppe, Aminogruppe (einzelnen oder Kombinationen).

Evtl. mehrere verschiedenen in der wässrigen Phase vorkommende Carbonsäuren können in Abhängigkeit des bei der Elektrolyse erzeugten pH-Wertes in der wässrigen Phase aus dieser in die andere Phase übertragen werden. So kann durch gezielte Wahl des pH-Wertes bei der Elektrolyse eine Selektion einer oder mehrerer Säuren aus mehreren Säuren erfolgen.

Der Erfindung liegt schliesslich die Aufgabe zugrunde, das vorbeschriebene Verfahren für das Recycling von Fermentationsrückständen einzusetzten, aus denen Zielmoleküle angereichert und Isoliert werden können.

Diese Aufgabe wird durch die kennzeichnenden Merkmale des Verwendungsanspruchs gelöst.

Die Erfindung betrifft daher die Verwendung des vorstehend beschriebenen Verfahrens zur Anreicherung und anschließenden Isolierung des/r vorstehend beschriebenen Zielmolekül(e), insbesondere von gesättigten oder ungesättigten Carbonsäuren mit wenigstens einer Carboxylgruppe, die ggf. substituiert ist, ggfs. mit sauerstoffhaltigen funktionellen Gruppen, bevorzugt durch eine pH-initiierte Ausfällung.

Ausführungsbeispiele der Erfindung werden nachfolgend beschrieben.

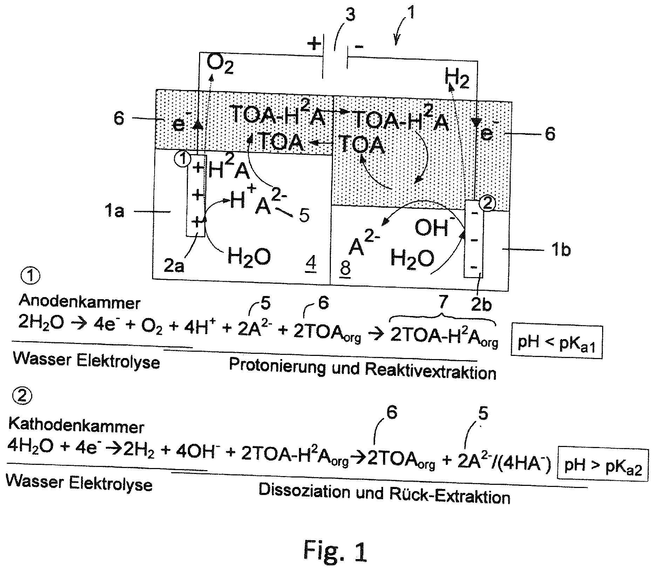

Die Figur 1 zeigt eine Elektrolyseeinheit 1 mit einer Anodenkammer 1a und einer Kathodenkammer 1b. Die beiden Kammern sind hinsichtlich ihrer Volumina getrennt, sodass kein unbeabsichtigter konvektiver Austausch der Phasen zwischen den beiden Kammern stattfinden kann. Die Kammern sind jedoch zum Ladungsträgerausgleich miteinander verbunden, sodass über die Anode 2a, die Kathode 2b und die Stromversorgung 3 eine Elektrolyse durchgeführt werden kann.

In der Anodenkammer 1a befindet sich eine wässrige erste Phase 4 die eine in Wasser gelöste Säure 5 umfasst. Bei der Durchführung der Elektrolyse entstehen an der Anode Wasserstoffionen (Protonen), sodass hierdurch in der wässrigen ersten Phase 4 eine Protonierung der Säure 5 erfolgt, sodass diese sich an das Reaktivextraktionsmittel 6 durch Wasserstoffbrückenbindung anlagert, welches gleichzeitig die zweite Phase 6 bildet oder zumindest in dieser enthalten ist. In dieser Darstellung sind die beiden Phasen übereinander liegend und somit aufeinander aufschwimmend dargestellt, was lediglich symbolischen Charakter zur Visualisierung der Phasen hat. Erfindungsgemäß bevorzugt ist es, die beiden Phasen 4 und 6 für die durchzuführende Extraktion zu dispergieren, z.B. in der Anodenkammer 1a oder auch schon zuvor.

Durch die Anlagerung mittels der Wasserstoffbrückenbindung entsteht demnach in der zweiten Phase 6 der Komplex 7 gemäß der ersten Gleichung in der Figur 1. Die erste Phase 4 kann beispielsweise einem Fermentationsreaktor entnommen sein, um die durch Fermentation gebildete Säure aus dieser Phase zu extrahieren. Durch die Elektrolyse erfolgt eine pH-Wert-Verschiebung unter die Säurezahl dieser Säure.

Die Figur 1 zeigt im oberen Bereich der beiden Elektrodenkammern Austauschpfeile zwischen den sich gegenüberliegenden oberen Phasen von Anodenkammer 1a und Kathodenkammer 1b. Dies bedeutet nicht, dass diese beiden Kammern untereinander verbunden sind bezüglich eines möglichen Stoffaustausches, sondern dass es verfahrensgemäß vorgesehen sein kann, nach einer ersten Extraktion der Säure 5 in die Phase 6 diese Phase 6 z.B. konvektiv zu übertragen in die Kathodenkammer 1b, um dort eine Rückextraktion in eine wässrige Phase 8 vorzunehmen.

Es werden sodann in einem nachfolgenden oder gegebenenfalls auch parallel durchführbaren Schritt unter Durchführung der Elektrolyse in der Kathodenkammer 1b OH⁻ -Ionen erzeugt, die zu einer Deprotonierung und einem Aufbrechen der Wasserstoffbrückenbindungen in der Phase 6 führt, sodass die an das Reaktivextraktionsmittel 6 gebundene Säure 5 abgespalten und deprotoniert wird. Aufgrund einer höheren Löslichkeit der deprotonierten Säure 5 in der wässrigen Phase 8 der Kathodenkammer 1b geht die deprotonierte Säure in die Lösung über und ist dort gegenüber der wässrigen Phase 4 angereichert bzw. aufkonzentriert.

Die Phase 6 und somit das Reaktivextraktionsmittel kann nach der Abspaltung gemäß den Pfeilen in die Anodenkammer 1a zurücküberführt werden, um die beiden Verfahrensschritte zyklisch durchzuführen. Bei der Elektrolyse wird in der Kathodenkammer 1b der pH-Wert über die Säurezahl der wässrigen Lösung, d. h. der Phase 8 erhöht. Auch in der Kathodenkammer ist es bevorzugt vorgesehen die beiden Phasen 6 und 8 zu dispergieren, um eine möglichst große Oberfläche zwischen den Phasen zu erhalten.

In der Kathodenkammer findet die Reaktion gemäß der Gleichung 2 statt, aus der ersichtlich ist, dass nach der die Dissoziation des gebildeten Komplexes 7 durchführenden Elektrolyse in dieser Kammer die Phase 6 im Wesentlichen dem ursprünglichen Reaktivextraktionsmittel entspricht.

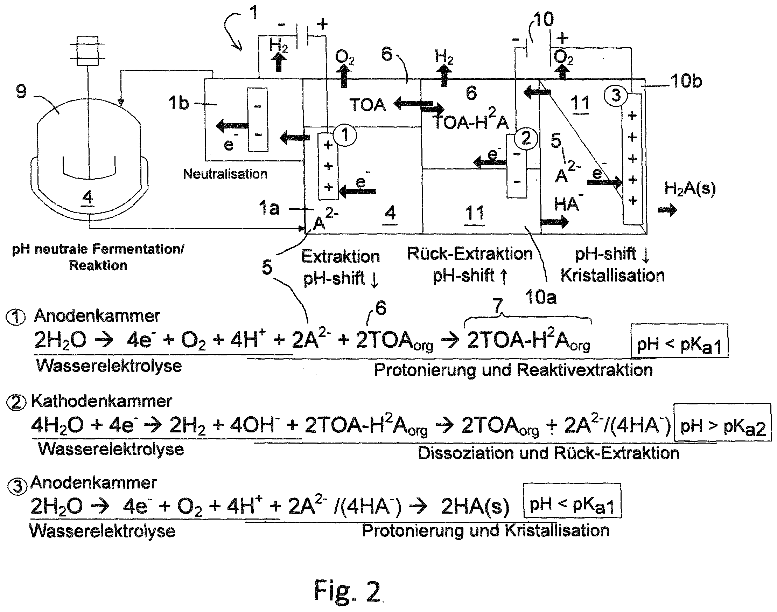

Die Figur 2 beschreibt eine weitere Ausführungsform des Verfahrens zur Extraktion und Auskristallisation einer Fermentationssäure aus einem Fermentationsreaktor 9. Dieser umfasst eine erste Phase 4, die durch Leitungen in die Anodenkammer 1a einer Elektrolyseeinheit 1 eingeleitet wird und in wässriger Lösung die Säure 5 umfasst. Wie auch bei der Ausführung der Figur 1 ist in der Anodenkammer 1a als zweite Phase 6 das Reaktivextraktionsmittel 6 vorgesehen, d. h. diese Phase wird überwiegend, wenn nicht insgesamt, durch das reine Reaktivextraktionsmittel 6 gebildet.

In der Anodenkammer 1a findet auch bei der Figur 2 exakt die Protonierungsreaktion statt die zu Figur 1 beschrieben ist und durch die Gleichung 1 unter der Figur 2 visualisiert ist, d. h. die Säure 5 wird durch Protonierung über Wasserstoffbrückenbindungen an das Reaktivextraktionsmittel 6 angelagert, um auch hier wiederum den durch Wasserstoffbrückenbindungen gebildeten Komplex 7 auszubilden.

Nach dieser ersten Extraktion der Säure 5 in die zweite Phase 6 visualisiert die Figur 2, dass die erste Phase 4, aus der die Säure extrahiert ist, über die Kathodenkammer 1b zurück in den Fermentationsreaktor 4 geführt wird, wobei die Elektrolyse in der Kathodenkammer 1b bewirkt, dass der bei der Protonierungsreaktion abgesenkte pH-Wert der wässrigen Phase 4 vor der Rückführung in den Fermentationsbehälter 9 durch die OH⁻- Ionen in der Kathodenkammer angepasst wird, d. h. angehoben wird, bevorzugt auf den pH-Wert der im Fermentationsbehälter 9 vorliegt. So kann die erste Phase 4 im Kreislauf zwischen dem Fermentationsbehälter 9 und der Anodenkammer 1a geführt werden.

Der zweite Schritt des hier beschriebenen Verfahrens sieht vor, dass die Phase 6, die nunmehr im Wesentlichen aus dem Komplex 7 besteht oder diesen zumindest überwiegend umfasst, in eine Kathodenkammer 10a einer zweiten Elektrolyseeinheit 10 überführt wird.

In dieser Kathodenkammer 10a befindet sich die überführte Phase 6 sowie eine weitere Phase 11, beispielsweise Wasser oder zumindest eine wässrige Phase die Wasser umfasst. Durch die hier stattfindende OH⁻-Ionenfreisetzung erfolgt eine Abspaltung der Säure 5 aus dem Komplex 7 und eine Deprotonierung der in dem Komplex gebundenen Säure, sodass die deprotonierte Säure 5 aufgrund besserer Löslichkeit im Wasser im Vergleich zur protonierten Säure in die Phase 11 überführt und dort angereichert, d. h. aufkonzentriert wird. Die Phase 11 bildet somit eine wässrige Phase in welcher die Säure 5 gegenüber der wässrigen Phase 4 angereichert ist.

Die Figur 2 visualisiert, dass die Phase 6 nach dem Aufbrechen des Komplexes 7 wieder in die Anodenkammer 1a der Elektrolyseeinheit 1 zurückgeführt wird, um dort den Extraktionsprozess wiederholt mit dieser Phase durchführen zu können. Die mit der Säure 5 aufkonzentrierte Phase 11 wird aus der Kathodenkammer 10a der Elektrolyseeinheit 10 in deren Anodenkammer 10b überführt, wo in dieser wässrigen Phase 11 eine pH-Wert Verschiebung zu geringeren pH-Werten aufgrund der Wasserstoffionenfreisetzung in dieser Kammer erfolgt. Da die Löslichkeit der Säure 5 in der wässrigen Phase 11 pH-Wert abhängig ist und insbesondere bei geringeren pH-Werten eine geringere Löslichkeit aufweist als bei höheren pH-Werten, führt die pH-Wert Absenkung in der Anodenkammer zu einem Auskristallisieren der Säure.

Die Säure kann somit als Feststoff aus der Anodenkammer 10b entnommen werden und beispielsweise ihrer gewünschten Anwendung zugeführt werden.

Die Figur 2 visualisiert, dass die Phase 11 nach dem Prozess der Auskristallisation wieder in die Kathodenkammer der zweiten Elektrolyseeinheit 10 zurücküberführt werden kann, um eine Rückextraktion wie zuvor beschrieben im Zyklus durchzuführen.

So wird deutlich, dass die jeweiligen Phasen 4, 6 und 11 jeweils im Kreislauf geführt werden können. Die Extraktion, Rückextraktion und Auskristallisierung können somit kontinuierlich durchgeführt werden.

Die Rückextraktion in der Kathodenkammer 10a erfolgt gemäß Gleichung 2 unter der Figur 2 und somit identisch wie zu Figur 1 beschrieben. Die Auskristallisation in der Anodenkammer wird durch Gleichung 3 der Figur 2 beschrieben.

Im nachfolgenden Beispiel wird die elektrochemisch initiierte Rückextraktion von Itaconsäure aus Trioctylamin beschrieben und die Durchführbarkeit belegt.

### Versuchsbeschreibung:

Für die Rückextraktion werden 20ml mit Itaconsäure beladenes TOA und 130ml 1M KCl Lösung in die Kathodenkammer sowie 130ml 1M KCl Lösung in die Anodenkammer gegeben. Als Anode wird ein Titanblech mit 7,5cm² und als Kathode ein Nickelblech mit 7,5cm² aktiver Fläche verwendet. Beide Kammern werden durch einen porösen Glasfilter konvektiv voneinander getrennt. Vor Beginn der Elektrolyse wird das System 25min dispergiert, um die Anfangskonzentration zu bestimmen. Vor Beginn der Elektrolyse wird in der wässrigen Phase der Kathodenkammer eine Konzentration von 17,3g/l Itaconsäure und ein pH Wert von 3,5 gemessen. Anschließend wird durch das Anlegen einer Spannung von 15-25V ein Stromfluss von 0,75A für 60min erzeugt. Nach Abschluss der Elektrolyse beträgt die Konzentration Itaconsäure in wässrigen Phase der Kathodenkammer 24,42 g/l und der pH-Wert 4,3. In dem nicht optimierten Versuchsaufbau wurde eine Faraday Effizienz der Extraktion von 51% der extrahierten Stoffmenge Itaconsäure bezogen auf die durch die übertragene Ladungsträgermenge maximal extrahierbare Stoffmenge Säure erzielt. Die Konzentration der Itaconsäure wurde mittels HPLC (Agilent Technologies 1100) auf einer Organic Acid Resin Säule mit RI und DAD Detektor gemessen.

Im nachfolgenden Beispiel wird die elektrochemisch initiierte Rückextraktion von Bernsteinsäure mit Trioctylamin-1-Octanol beschrieben und die Durchführbarkeit belegt.

### Extraktion Bernsteinsäure:

Für die Extraktion werden 160g 0,5M K2SO4 Lösung mit 10g Bernsteinsäure und einem durch KOH eingestellten pH-Wert von 7 in die Anoden Kammer gegeben. Gleichzeitig werden 200g 0,5M K2SO4 Lösung mit 1,5g Bernsteinsäure in die Kathodenkammer gegeben. Anschließend werden 35g Trioctylamin-1-Octanol mit einem Verhältnis von 0,4:0,6 %m in die Anodenkammer gegeben und durch Rühren mit der wässrigen Phase in Kontakt gebracht. Als Anode wird eine platinbeschichtete Titanelektrode mit 7,5cm² und als Kathode ein Nickelblech mit 7,5cm² aktiver Fläche verwendet. Beide Kammern werden durch einen porösen Glasfilter konvektiv voneinander getrennt. Vor Beginn der Elektrolyse wird das System 25min dispergiert, um die Anfangskonzentration zu bestimmen. Vor Beginn der Elektrolyse wird in der wässrigen Phase der Anodenkammer eine Konzentration von 45,11g/l Bernsteinsäure und ein pH-Wert von 7 gemessen.

Anschließend wird durch das Anlegen einer Spannung von 15-25V ein Stromfluss von 0,57A für 325min erzeugt. Nach Abschluss der Elektrolyse beträgt die Konzentration der Bernsteinsäure in der wässrigen Phase der Anodenkammer 33,40 g/l und der pH-Wert 4,84. In dem nicht optimierten Versuchsaufbau wurde eine Faraday Effizienz der Extraktion von 61% der protonierten Stoffmenge Bernsteinsäure bezogen auf die durch die übertragene Ladungsträgermenge maximal protonierbare Stoffmenge Säure erzielt. Die Konzentration der Bernsteinsäure wurde mittels HPLC (Agilent Technologies 1100) auf einer Organic Acid Resin Säule mit RI und DAD Detektor gemessen.

### Rückextraktion:

Für die Rückextraktion werden 40g mit Bernsteimsäure beladenes Trioctylamin-1-Octanol mit einem Verhältnis von 0,4:0,6 %m und 160g 0,5M K2SO4 Lösung in die Kathodenkammer sowie 200g 0,5M K2SO4 Lösung in die Anodenkammer gegeben. Als Anode wird eine platinbeschichtete Titanelektrode mit 7,5cm² und als Kathode ein Nickelblech mit 7,5cm² aktiver Fläche verwendet. Beide Kammern werden durch einen porösen Glasfilter konvektiv voneinander getrennt. Vor Beginn der Elektrolyse wird das System 25min dispergiert, um die Anfangskonzentration zu bestimmen. Vor Beginn der Elektrolyse wird in der wässrigen Phase der Kathodenkammer eine Konzentration von 22,31g/l Bernsteinsäure und ein pH-Wert von 2,76 gemessen. Anschließend wird durch das Anlegen einer Spannung von 15-20V ein Stromfluss von 0,4A für 280min erzeugt. Nach Abschluss der Elektrolyse beträgt die Konzentration der Bernsteinsäure in der wässrigen Phase der Kathodenkammer 32,53g/l und der pH-Wert 6,3.

Die Funktionalität wird an Hand zweier Versuche zur Kristallisation von Dicarbonsäuren (Itakonsäure und Bernsteinsäure) gezeigt.

Für die Itaconsäure-Kristallisation wurde eine Itaconsäurelösung mit pH Wert 4,1 durch Zugabe von Kaliumlauge (50 Gew.-%) bei einer Beladung von 0,32 g_{IA} / g_{H2O} angesetzt. Anschließend wurde Elektrolyt (El) K₂SO₄ zugegeben, sodass eine Beladung von 0,07 g_{EL} / g_{H2O} in der Phase vorlag. 180 ml der wässrigen Phase wurde mit einem Rührer homogen für 8,5 h durchmischt. Die Elektrolyse fand mit einer Mixed Metal Anode aus Titan mit einem Rutheniumoxid Coating der Firma Magento Special Anodes B.V. und einer Nickel Kathode bei einer Spannung von 20-25 V statt. Die Temperatur wurde mit dem Thermostat Lauda E100 konstant auf 15 °C gehalten. Es wird die Masse an kristalliner Itaconsäure und eine optische Aufnahme der Kristalle im Durchlicht mit dem Mikroskop Olympus BH-2 mit der Kamera Olympus DP25 und deren Reinheit zur Überprüfung der Funktionalität herangezogen. Zusätzlich werden Effizienzparameter des elektrochemischen Systems angegeben.

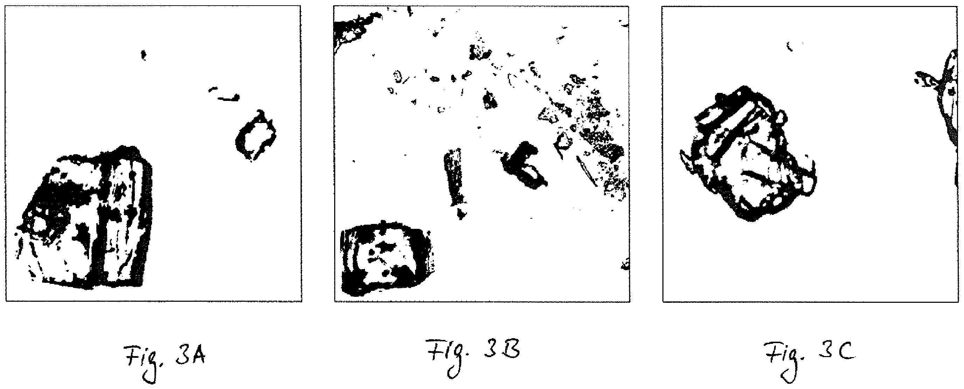

Ergebnis: Es konnten 2,65 g_{IA} gewonnen werden. Durchlicht Mikroskop Aufnahmen sind in Figur 3 gezeigt. Figur 3A zeigt ein Kristallisat 40-fach vergrößert, die Figuren 3B und 3c zeigen ein Kristallisat 60-fach vergrößert. Die spezifische Stromstärke betrug im Mittel 0,025 A / cm² bei einem Anodenspezifischem Umsatz von 0,031 g/ cm² h. Eine Faradayeffizienz von 43,74 % konnte dabei erzielt werden.

Für die Bernsteinsäure Kristallisation wurde eine Bernsteinsäurelösung mit pH Wert 4,144 durch Zugabe von Kaliumlauge (50 Gew.-%) bei einer Beladung von 0,187 g_{SA} / g_{H2O} angesetzt. Anschließend wurde Elektrolyt (El) K₂SO₄ zugegeben, sodass eine Beladung von 0,033 g_{EL} / g_{H2O} in der Phase vorlag. 300 ml der wässrigen Phase wurde mit einem Rührer homogen für 2 h durchmischt. Die Elektrolyse fand mit einer Mixed Metal Anode aus Titan mit einem Rutheniumoxid Coating der Firma Magento Special Anodes B.V. und einer Nickel Kathode bei einer Spannung von 20 V statt. Die Temperatur wurde mit dem Thermostat Lauda E100 konstant auf 15 °C gehalten. Es wird die Masse an kristalliner Bernsteinsäure und eine optische Aufnahme der Kristalle im Durchlicht mit dem Mikroskop Olympus BH-2 mit der Kamera Olympus DP25 und deren Reinheit zur Überprüfung der Funktionalität herangezogen. Zusätzlich werden Effizienzparameter des elektrochemischen Systems angegeben.

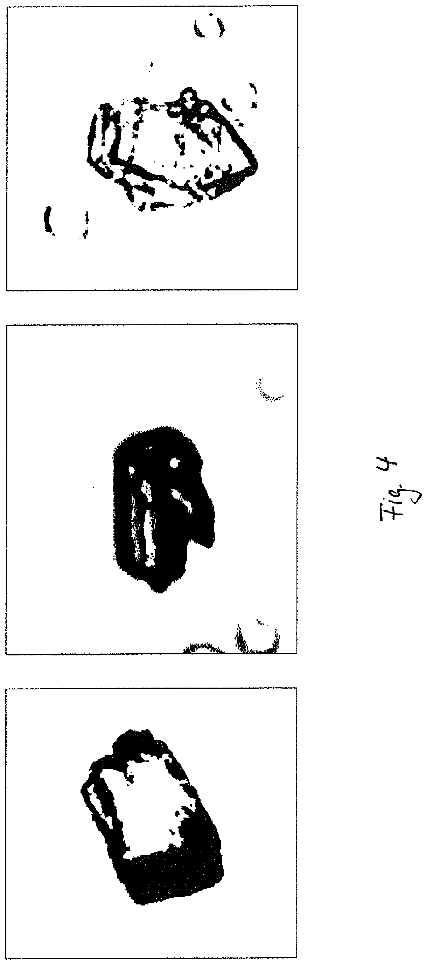

Ergebnis: Es konnten 0,962 g_{IA} gewonnen werden. Durchlicht Mikroskop Aufnahmen sind in Figur 4 gezeigt. Diese zeigt das Bernsteinsäure-Kristallisat mit 40-facher Vergrößerung. Die gewonnene Reinheit der Kristalle entspricht 89,4%. Die spezifische Stromstärke betrug im Mittel 0,034 A / cm² bei einem Anodenspezifischem Umsatz von 0,048 g/ cm² h. Eine Faradayeffizienz von 32,34 % konnte dabei erzielt werden.

## Patentansprüche

1. Verfahren zur Übertragung eines Zielstoffes (5) zwischen zwei flüssigen Phasen (4, 6; 6, 8; 6,11), von denen wenigstens eine Phase (4, 6) den zu übertragenden Zielstoff (5) umfasst und wenigstens eine Phase (4, 8, 11) eine wässrige Phase ist, wobei zumindest die wässrige Phase (4, 8, 11) in einer von zwei durch Ladungsträgeraustausch elektrisch leitfähig verbundenen und hinsichtlich ihrer Volumina getrennten Elektrodenkammern (1a, 1b, 10a, 10b) angeordnet ist, oder wobei die Phasen (4, 6; 6, 8; 6,11) gemeinsam in einer von zwei elektrisch leitfähig verbundenen und hinsichtlich ihrer Volumina getrennten Elektrodenkammern (1a, 1b, 10a, 10b) angeordnet sind und durch die bei der Elektrolyse entstehenden H- und/oder OH-Ionen in der wässrigen Phase (4, 8, 11) eine pH-Wert-Änderung erzeugt wird, welche einen Übertragungsprozess des Zielstoffes (5) zwischen den Phasen (4, 6; 6, 8; 6,11) initiiert, wenn sich die Phasen kontaktieren oder in Kontakt gebracht werden, **dadurch gekennzeichnet, dass**
a. in einem ersten Verfahrensschritt eine erste Phase (4) durch eine wässrige Lösung einer Säure (5) als Zielmolekül (5) gebildet wird und eine zweite Phase (6)
i. ein Reaktivextraktionsmittel als Bindungspartner (6) umfasst, und/oder
ii. ein organisches Lösungsmittel (6) umfasst wobei in der Anodenkammer (1a) von beiden Elektrodenkammern (1a, 1b) die Säure (5) protoniert wird und
iii. reaktiv an das Reaktivextraktionsmittel (6) angelagert wird, oder
iv. in das organische Lösungsmittel (6) in Lösung geht, in welchem die protonierte Säure (5) eine höhere Löslichkeit hat im Vergleich zur deprotonierten Säure (5)
und hierdurch von der ersten Phase (4) in die zweite Phase (6) übertragen wird
b. in einem zweiten Verfahrensschritt eine erste Phase (6)
i. durch einen Komplex (7) eines Reaktivextraktionsmittels (6) als Bindungspartner (6) und einer daran gebundene protonierte Säure (5) als Zielmolekül (5) gebildet wird, oder
ii. durch ein organisches Lösungsmittel (6) gebildet wird, in welchem eine protonierte Säure (5) gelöst ist, oder
iii. durch eine Mischung aus Reaktivextraktionsmittel (6) und organischem Lösungsmittel (6) gebildet wird, wobei ein oder beide in i) oder ii) genannten Effekte auftreten
und die zweite Phase Wasser (8, 11) umfasst, wobei in der Kathodenkammer (10a) von beiden Elektrodenkammern (10a, 10b) die protonierte Säure (5) deprotoniert wird und
iv. durch das Aufbrechen der Bindungen vom Reaktivextraktionsmittel (6) abgespalten wird und/oder
v. in der wässrigen Phase (8, 11) in Lösung geht
und hierdurch von der ersten Phase (6) in die zweite Phase (8, 11) übertragen wird
c. wobei der zweite Verfahrensschritt b) zeitlich nach oder gleichzeitig mit dem ersten Verfahrensschritt a) durchgeführt wird und die zweite Phase (6) des ersten Verfahrensschrittes a) die erste Phase (6) des zweiten Verfahrensschrittes b) bildet.

2. Verfahren nach einem der vorherigen Ansprüche, **dadurch gekennzeichnet, dass** der Zielstoff (5) ein Zielmolekül (5) ist, welches ausgewählt ist aus der Gruppe der wasserlöslichen Aminosäuren und / oder wasserlöslichen Carbonsäuren.

3. Verfahren nach einem der vorherigen Ansprüche, **dadurch gekennzeichnet, dass** die am Stoffaustausch teilnehmende nicht wässrige Phase (6)
a. ein organisches Lösungsmittel (6) umfasst wobei das organische Lösungsmittel (6) gewählt ist aus mit der wässrigen Phase (4, 8, 11) nicht / nur teileweise mischbaren Alkoholen, Estern, Ketonen, aliphatischen & aromatischen Kohlenwasserstoffen, oder
b. einen reaktiven Bindungspartner (6) umfasst, der abhängig vom pH-Wert reversible Bindungen mit dem Zielstoff (5) eingehen kann, der ausgewählt ist aus der Gruppe der aliphatischen Aminen mit wenigstens 10 Kohlenstoffatomen, Organophosphorsäuren mit 2 bis 3 Alkylgruppen.

4. Verfahren nach einem der vorherigen Ansprüche, **dadurch gekennzeichnet, dass** es in einem dispergierten Zustand der beiden Phasen durchgeführt wird, insbesondere die Phasen (4, 6, 8, 11) in der Elektrodenkammer (1a, 1b, 10a, 10b) zu einer Dispersion durchmischt werden.

5. Verfahren nach einem der vorherigen Ansprüche, **dadurch gekennzeichnet, dass** die erste Phase (4) im ersten Verfahrensschritt a) einem Fermentationsreaktor (9) entnommen wird und nach Durchführung der Übertragung des Zielmoleküls (5) auf die zweite Phase (6) über eine der Anodenkammer (1a) elektrisch zugeordnete Kathodenkammer (1b) bei oder nach Durchführung der Elektrolyse in den Fermentationsreaktor (9) zurückgeführt wird.

6. Verfahren nach einem der vorherigen Ansprüche, **dadurch gekennzeichnet, dass** die zweite Phase (11) im zweiten Verfahrensschritt b) in eine Anodenkammer (10b) einer Elektrolyseeinheit (10) überführt wird, insbesondere in die Anodenkammer (10b), die der in zweiten Verfahrensschritt b) genannten Kathodenkammer (10a) elektrisch zugeordnet ist und durch Senkung des pH-Wertes in der zweiten Phase (11) mittels durchgeführter Elektrolyse aufgrund der pH-Wert-abhängigen Löslichkeit der Säure (5) in Wasser die Säure (5) auskristallisiert wird.

7. Verfahren nach Anspruch 6, **dadurch gekennzeichnet, dass** nach Auskristallisierung die von der auskristallisierten Säure (5) separierte zweite Phase (11) in die Kathodenkammer (10a) zurückgeführt wird.

8. Verfahren nach einem der vorherigen Ansprüche, **dadurch gekennzeichnet, dass** das Zielmolekül (5) durch die nacheinander durchgeführten Verfahrensschritte a) und b) aus einer wässrigen Phase (4) in eine wässrige Phase (8, 11) unter Erhöhung der Konzentration in der letzten wässrigen Phase (8, 11) überführt wird.

9. Verfahren nach einem der vorherigen Ansprüche, **dadurch gekennzeichnet, dass** die Anodenkammer (1a) und die Kathodenkammer (10a) Elektrodenkammern verschiedener Elektrolyseeinheiten (1, 10) sind.

10. Verfahren nach einem der vorherigen Ansprüche, **dadurch gekennzeichnet, dass** der bei einer jeweiligen Elektrolyse entstehende Sauerstoff für die Sauerstoffversorgung eines Fermentationsreaktors (9) genutzt wird.

11. Verfahren nach irgendeinem der vorherigen Ansprüche, **dadurch gekennzeichnet, dass** im Verfahrensschritt a) von Anspruch 1 der Bindungspartner (6) in der zweiten Phase (6) ausgewählt ist aus aliphatischen Aminen mit wenigstens 10 Kohlenstoffatomen.

## Claims

1. Method of transferring a target substance (5) between two liquid phases (4, 6; 6, 8; 6, 11), of which at least one phase (4, 6) comprises the target substance (5) to be transferred and at least one phase (4, 8, 11) is an aqueous phase, where at least the aqueous phase (4, 8, 11) is disposed in one of two electrode chambers (1a, 1b, 10a, 10b) that are separated in terms of their volumes and are electrically conductively connected by charge carrier exchange, or where the phases (4, 6; 6, 8; 6, 11) are collectively disposed in one of two electrically conductively connected electrode chambers (1a, 1b, 10a, 10b) that are separated in terms of their volumes, and a change in pH is generated in the aqueous phase (4, 8, 11) by the H and/or OH ions formed in the electrolysis, which initiates a process of transfer of the target substance (5) between the phases (4, 6; 6, 8; 6, 11) when the phases come into contact or are brought into contact, **characterized in that**
a. in a first method step, a first phase (4) is formed by an aqueous solution of an acid (5) as target molecule (5) and a second phase (6)
i. comprises a reactive extractant as binding partner (6) and/or
ii. comprises an organic solvent (6),
where the acid (5) is protonated in the anode chamber (1a) of both electrode chambers (1a, 1b) and
iii. is added reactively onto the reactive extractant (6) or
iv. goes into solution in the organic solvent (6), in which the protonated acid (5) has higher solubility compared to the deprotonated acid (5),
and is thereby transferred from the first phase (4) into the second phase (6),
b. in a second method step, a first phase (6)
i. is formed by a complex (7) of a reactive extractant (6) as binding partner (6) and a protonated acid (5) bound thereto as target molecule (5), or
ii. is formed by an organic solvent (6) in which a protonated acid (5) is dissolved, or
iii. is formed by a mixture of reactive extractant (6) and organic solvent (6), with occurrence of one or both effects mentioned in i) or ii),
and the second phase comprises water (8, 11), where the protonated acid (5) is deprotonated in the cathode chamber (10a) of the two electrode chambers (10a, 10b), and
iv. is split off from the reactive extractant (6) by the breaking of the bonds and/or
v. goes into solution in the aqueous phase (8, 11),
and is thereby transferred from the first phase (6) into the second phase (8, 11),
c. wherein the second method step b) is conducted after or simultaneously with the first method step a), and the second phase (6) of the first method step a) forms the first phase (6) of the second method step b).

2. Method according to one of the preceding claims, **characterized in that** the target substance (5) is a target molecule (5) selected from the group of the water-soluble amino acids and/or water-soluble carboxylic acids.

3. Method according to either of the preceding claims, **characterized in that** the nonaqueous phase (6) involved in the mass transfer
a. comprises an organic solvent (6), where the organic solvent (6) is selected from alcohols, esters, ketones, aliphatic & aromatic hydrocarbons that are immiscible/only partly miscible with the aqueous phase (4, 8, 11), or
b. comprises a reactive binding partner (6) which, depending on the pH, can enter into reversible bonds with the target substance (5), selected from the group of the aliphatic amines having at least 10 carbon atoms, organophosphorus acids having 2 to 3 alkyl groups.

4. Method according to any of the preceding claims, **characterized in that** it is conducted in a dispersed state of the two phases, especially **in that** the phases (4, 6, 8, 11) are mixed in the electrode chamber (1a, 1b, 10a, 10b) to give a dispersion.

5. Method according to any of the preceding claims, **characterized in that** the first phase (4) in the first method step a) is withdrawn from a fermentation reactor (9) and, after performance of the transfer of the target molecule (5) to the second phase (6), is recycled into the fermentation reactor (9) via a cathode chamber (1b) electrically assigned to the anode chamber (1a) on or after performance of the electrolysis.

6. Method according to any of the preceding claims, **characterized in that** the second phase (11) in the second method step b) is transferred into an anode chamber (10b) of an electrolysis unit (10), especially into the anode chamber (10b) electrically assigned to the cathode chamber (10a) mentioned in the second method step b), and the acid (5) is crystallized by lowering the pH in the second phase (11) by performance of electrolysis on account of the pH-dependent solubility of the acid (5) in water.

7. Method according to Claim 6, **characterized in that,** after crystallization, the second phase (11) separated from the crystallized acid (5) is recycled into the cathode chamber (10a).

8. Method according to any of the preceding claims, **characterized in that** the target molecule (5) is transferred by the successively conducted method steps a) and b) from an aqueous phase (4) into an aqueous phase (8, 11) with an increase in the concentration in the last aqueous phase (8, 11).

9. Method according to any of the preceding claims, **characterized in that** the anode chamber (1a) and the cathode chamber (10a) are electrode chambers of different electrolysis units (1, 10).

10. Method according to any of the preceding claims, **characterized in that** the oxygen formed in a respective electrolysis is utilized for supply of oxygen to a fermentation reactor (9).

11. Method according to any one of the preceding claims, **characterized in that,** in method step a) of Claim 1, the binding partner (6) in the second phase (6) is selected from aliphatic amines having at least 10 carbon atoms.

## Revendications

1. Procédé destiné à transférer une substance cible (5) entre deux phases liquides (4, 6 ; 6, 8 ; 6, 11), dont au moins une phase (4, 6) comprend la substance cible (5) à transférer et au moins une phase (4, 8, 11) est une phase aqueuse, au moins la phase aqueuse (4, 8, 11) étant agencée dans l'une de deux chambres d'électrode (1a, 1b, 10a, 10b) reliées de manière électriquement conductrice par échange de supports de charges et séparées en ce qui concerne leurs volumes, ou les phases (4, 6 ; 6, 8 ; 6, 11) étant agencées conjointement dans l'une de deux chambres d'électrode (1a, 1b, 10a, 10b) reliées de manière électriquement conductrice et séparées en ce qui concerne leurs volumes et les ions H et/ou OH formés lors de l'électrolyse générant une modification de valeur de pH dans la phase aqueuse (4, 8, 11), qui initie un processus de transfert de la substance cible (5) entre les phases (4, 6 ; 6, 8 ; 6, 11), lorsque les phases entrent ou sont mises en contact, **caractérisé en ce que**
a. dans une première étape de procédé, une première phase (4) est formée par une solution aqueuse d'un acide (5) en tant que molécule cible (5) et une deuxième phase (6)
i. comprend un agent d'extraction réactif en tant que partenaire de liaison (6) et/ou
ii. comprend un solvant organique (6) l'acide (5) étant protoné dans la chambre anodique (1a) des deux chambres d'électrode (1a, 1b) et
iii. fixé de manière réactive à l'agent d'extraction réactif (6) ou
iv. passant en solution dans le solvant organique (6) dans lequel l'acide protoné (5) présente une solubilité supérieure par rapport à celle dans l'acide déprotoné (5)
et étant ainsi transféré de la première phase (4) dans la deuxième phase (6)
b. dans une deuxième étape de procédé, une première phase (6)
i. est formée par un complexe (7) d'un agent d'extraction réactif (6) en tant que partenaire de liaison (6) et d'un acide protoné (5) lié à celui-ci en tant que molécule cible (5) ou
ii. est formée par un solvant organique (6) dans lequel un acide protoné (5) est dissous ou
iii. est formée par un mélange d'agent d'extraction réactif (6) et de solvant organique (6), l'un ou les deux effets mentionnés dans i) ou ii) se produisant
et la deuxième phase comprend de l'eau (8, 11), l'acide protoné (5) étant déprotoné dans la chambre cathodique (10a) des deux chambres d'électrode (10a, 10b) et
iv. étant dissocié de l'agent d'extraction réactif (6) par la rupture des liaisons et/ou
v. passant en solution dans la phase aqueuse (8, 11)
et étant ainsi transféré de la première phase (6) dans la deuxième phase (8, 11)
c. la deuxième étape de procédé b) étant effectuée temporellement après ou simultanément avec la première étape de procédé a) et la deuxième phase (6) de la première étape de procédé a) formant la première phase (6) de la deuxième étape de procédé b) .

2. Procédé selon l'une des revendications précédentes, **caractérisé en ce que** la substance cible (5) est une molécule cible (5) qui est choisie dans le groupe des acides aminés solubles dans l'eau et/ou des acides carboxyliques solubles dans l'eau.

3. Procédé selon l'une des revendications précédentes, **caractérisé en ce que** la phase (6) non aqueuse participant à l'échange de substances
a. comprend un solvant organique (6), le solvant organique (6) étant choisi parmi les alcools, esters, cétones, hydrocarbures aliphatiques et aromatiques non miscibles/uniquement partiellement miscibles avec la phase aqueuse (4, 8, 11) ou
b. comprend un partenaire de liaison réactif (6) qui peut former, en fonction du pH, des liaisons réversibles avec la substance cible (5), qui est choisi dans le groupe des amines aliphatiques comprenant au moins 10 atomes de carbone, les acides organophosphoriques comprenant 2 à 3 groupes alkyle.

4. Procédé selon l'une des revendications précédentes, **caractérisé en ce qu'**il est mis en œuvre dans un état dispersé des deux phases, en particulier les phases (4, 6, 8, 11) sont mélangées en une dispersion dans la chambre d'électrode (1a, 1b, 10a, 10b).

5. Procédé selon l'une des revendications précédentes, **caractérisé en ce que** la première phase (4) dans la première étape de procédé a) est soutirée d'un réacteur de fermentation (9) et est recyclée dans le réacteur de fermentation (9) après la mise en œuvre du transfert de la molécule cible (5) à la deuxième phase (6) par l'intermédiaire d'une chambre cathodique (1b) électriquement associée à la chambre anodique (1a), lors de la mise en œuvre de l'électrolyse ou après celle-ci.

6. Procédé selon l'une des revendications précédentes, **caractérisé en ce que** la deuxième phase (11), dans la deuxième étape de procédé b), est transférée dans une chambre anodique (10b) d'une unité d'électrolyse (10), en particulier dans la chambre anodique (10b) qui est associée électriquement à la chambre cathodique (10a) mentionnée dans la deuxième étape de procédé b) et l'acide (5) est séparé par cristallisation par la diminution de la valeur du pH dans la deuxième phase (11) au moyen de l'électrolyse mise en œuvre en raison de la solubilité dépendant du pH de l'acide (5) dans l'eau.

7. Procédé selon la revendication 6, **caractérisé en ce que** la deuxième phase (11) séparée de l'acide (5) séparé par cristallisation après la séparation par cristallisation est recyclée dans la chambre cathodique (10a).

8. Procédé selon l'une des revendications précédentes, **caractérisé en ce que** la molécule cible (5) est transférée par les étapes de procédé a) et b) mises en œuvre successivement à partir d'une phase aqueuse (4) dans une phase aqueuse (8, 11) avec augmentation de la concentration dans la dernière phase aqueuse (8, 11).

9. Procédé selon l'une des revendications précédentes, **caractérisé en ce que** la chambre anodique (1a) et la chambre cathodique (10a) sont des chambres d'électrode de différentes unités d'électrolyse (1, 10).

10. Procédé selon l'une des revendications précédentes, **caractérisé en ce que** l'oxygène formé lors d'une électrolyse respective est utilisé pour l'alimentation en oxygène d'un réacteur de fermentation (9).

11. Procédé selon l'une quelconque des revendications précédentes, **caractérisé en ce que**, dans l'étape de procédé a) de la revendication 1, le partenaire de liaison (6) dans la deuxième phase (6) est choisi parmi les amines aliphatiques comprenant au moins 10 atomes de carbone.
